(19) Europäisches Patentamt
European Patent Office
Office européen des brevets

(11) Veröffentlichungsnummer: **0 147 777**
**A2**

# (12) EUROPÄISCHE PATENTANMELDUNG

(21) Anmeldenummer: **84115773.8**

(22) Anmeldetag: **19.12.84**

(51) Int. Cl.⁴: **C 07 H 13/00**
C 07 H 15/00, A 61 K 31/00

(30) Priorität: **30.12.83 DE 3347522**

(43) Veröffentlichungstag der Anmeldung:
**10.07.85 Patentblatt 85/28**

(84) Benannte Vertragsstaaten:
**AT BE CH DE FR GB IT LI NL SE**

(71) Anmelder: **Troponwerke GmbH & Co. KG**
**Berliner Strasse 156**
**D-5000 Köln 80(DE)**

(72) Erfinder: **Lockhoff, Oswald, Dr.**
**Morgengraben 14**
**D-5000 Köln 80(DE)**

(72) Erfinder: **Stadler, Peter, Dr.**
**Im Ideck 8**
**D-5657 Haan 1(DE)**

(72) Erfinder: **Opitz, Hans-Georg z.Zt. Miles Lab., Inc.**
**4th & Parker Streets P.O. Box 1986**
**Berkeley, California 94701(US)**

(72) Erfinder: **Horstmann, Harald, Dr.**
**Claudiusweg 19**
**D-5600 Wuppertal 1(DE)**

(72) Erfinder: **Junge, Bodo, Dr.**
**Spiekern 23**
**D-5600 Wuppertal 23(DE)**

(72) Erfinder: **Pelster, Bernhard, Dr.**
**Pleisufer 6a**
**D-5205 St. Augustin 1(DE)**

(74) Vertreter: **Adrian, Albert, Dr. et al,**
**c/o BAYER AG Konzernverwaltung RP Patentabteilung**
**D-5090 Leverkusen 1, Bayerwerk(DE)**

(54) **N-glycosylierte Carbonsäureamid-Derivate als Mittel bei der Bekämpfung von Erkrankungen des rheumatischen Formenkreises.**

(57) Die vorliegende Erfindung betrifft die Verwendung der Verbindungen der allgemeinen Formel I

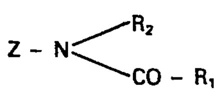

I

als Mittel bei der Bekämpfung von Erkrankungen des rheumatischen Formenkreises. In der allgemeinen Formel I bedeutet $R_1$ Wasserstoff oder einen gegebenenfalls substituierten geradkettigen oder verzweigten, gesättigten oder einfach bzw. mehrfach ungesättigten Alkylrest mit einem bis zu 30 Kohlenstoffatomen, wobei dieser Rest $R_1$ auch durch bis zu 5, vorzugsweise 1 oder 2 O, S und/oder N unterbrochen sein kann, mit der Maßgabe, daß $-CO-R_1$ keine Acylgruppe mit 1 - 5 C-Atomen darstellt, wenn $R_2$ Alkyl mit 10 - 20 C-Atomen bedeutet.

EP 0 147 777 A2

TROPONWERKE GmbH & Co. KG

5000 Köln 80

Je/div-c

## N-glycosylierte Carbonsäureamid-Derivate als Mittel bei der Bekämpfung von Erkrankungen des rheumatischen Formenkreises

Die vorliegende Erfindung betrifft die Verwendung der Verbindungen der allgemeinen Formel I

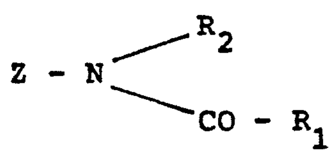

I

als Mittel bei der Bekämpfung von Erkrankungen des rheumatischen Formenkreises. In der allgemeinen Formel I bedeutet $R_1$ Wasserstoff oder einen gegebenenfalls substituierten geradkettigen oder verzweigten, gesättigten oder einfach bzw. mehrfach ungesättigten Alkylrest mit einem bis zu 30 Kohlenstoffatomen, wobei dieser Rest $R_1$ auch durch bis zu 5, vorzugsweise 1 oder 2 O, S und/oder N unterbrochen sein kann, mit der Maßgabe, daß $-COR_1$ keine Acylgruppe mit 1 - 5 C-Atomen darstellt, wenn $R_2$ Alkyl mit 10 - 20 C-Atomen bedeutet.

Bei einer Unterbrechung der Kette durch N trägt dieser Stickstoff entweder H oder einen $C_1$-$C_{20}$-, vorzugsweise $C_1$-$C_5$-Alkylrest oder einen -CO-Alkylrest, wobei diese Alkylgruppe 1 - 20, vorzugsweise 1 - 5 C-Atome aufweist.

Bevorzugt stellt $R_1$ einen Alkyl- oder Alkenylrest mit 1 bis 21 Kohlenstoffatomen vorzugsweise mit 9 bis 21 C-Atomen dar. Beispielhaft für gesättigte Reste seien hier genannt Methyl, Ethyl, Propyl, i-Propyl, Butyl, i-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Docosyl, Ethylpentyl, Methyldecyl, i-Propyldecyl, Methyltridecosyl.

Ungesättigte Reste sind beispielsweise Ethylen, Propenyl-1, Propenyl-2, i-Butenyl, Butenyl-1, Butenyl-2, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Decenyl, 5-Decenyl, 9-Decenyl, 8-Heptadecenyl, 1,3-Butadienyl, 1,3-Pentadienyl, 1,4-Pentadienyl, 2,4-Pentadienyl, 8,11-Heptadecandienyl, 8,11,14-Heptadecantrienyl. Im allgemeinen sind die längerkettigen, ungesättigten Reste bevorzugt, speziell die ein- oder zweifach ungesättigten Alkenyle mit 7-21 C-Atomen.

Die ungesättigten Kohlenwasserstoffreste können dabei als reine cis- oder trans-Isomere oder auch als Isomerengemische vorliegen.

Beispiele für Fälle in denen die Kohlenwasserstoffreste $R_1$ in Formel I durch O, S und N bzw. entsprechende Atomgruppierungen unterbrochen oder durch diese Atome enthaltende Gruppen oder durch Halogenatome substituiert werden, sind Methoxyethyl-, 2-(2-Methoxyethoxy)-ethyl-, 2-/2-(2-Methoxyethoxy)-ethoxy/-ethyl-, Hydroxyheptadecenyl-,

Oxobutyl-, Amino-decyl-, N-Methyl-aminodecyl-, Fluormethyl-, ß-Hydroxytridecyl- oder Mercaptoethylrest.

Die Kohlenwasserstoffreste $R_1$ in Formel I können auch Phenylreste enthalten, wobei diese Phenylreste gegebenenfalls auch durch einen bis drei Substituenten aus der Reihe Nitro, Niedrigalkyl oder durch 1 - 5 Halogenatome substituiert sein können.

$R_2$ in Formel I steht für Wasserstoff oder einen geradkettigen oder verzweigten, gesättigten oder einfach bzw. mehrfach ungesättigten Alkyl-, Cycloalkyl-, Alkylcycloalkyl- oder einen Aralkylrest mit bis zu 30 Kohlenstoffatomen, wobei im Rest $R_2$ auch einzelne Methylen- oder Methingruppen durch bis zu 5 Sauerstoff- oder Schwefelatome oder N, NH oder N-Niederalkylgruppierungen ersetzt sein können. Einzelne Wasserstoffatome in den Alkyl, Cycloalkyl oder Aralkylresten können auch durch bis zu 5 Sauerstoff enthaltene Gruppen oder Halogenatome substituiert sein.

Beispiele, in welchen $R_2$ in Formel I für einen geradkettigen oder verzweigten, gegebenenfalls einfach oder mehrfach ungesättigten Alkylrest steht, sind die für $R_1$ genannten.

Besonders seien genannt Methyl, Propyl, Hexyl, Decyl, Undecyl, Dodecyl, Tridecyl, Tetradecyl, Pentadecyl, Hexadecyl, Heptadecyl, Octadecyl, Nonadecyl, Docosyl, Myricyl, Ethylhexyl, Isobutyl, Propenyl, Octenyl, Hexadienyl, Docosenyl, Dimethylhexenyl und 2-(Cyclohexyl)-ethyl. Die

ungesättigten Kohlenwasserstoffreste können als reine cis- oder trans-Isomere oder als Isomerengemisch vorliegen. Durch Sauerstoffatome enthaltende Gruppen substituierte Kohlenwasserstoffreste $R_2$ sind beispielsweise Hydroxypropyl und Hydroxydimethyloctyl, durch Sauerstoffatome unterbrochene Kohlenwasserstoffreste sind beispielsweise Alkoxyalkylreste oder (Alkoxy-alkoxy)-alkylreste wie Metoxybutyl oder Butoxypropyl oder Ethoxyethoxyethyl, ein durch N und O unterbrochener Rest ist beispielsweise 2-(N-morpholino)-ethyl und als Beispiel für einen halogensubstituierten Kohlenwasserstoffrest sei Trifluormethylethyl genannt.

Aralkyl für $R_2$ in Formel I ist zum Beispiel Aryl-niedrigalkyl wie Benzyl, Phenethyl oder Phenylhexyl, wobei der Phenylkern gegebenenfalls einfach oder mehrfach, vorzugsweise ein- oder zweifach, zum Beispiel durch Niederalkyl, Trifluormethyl, Halogen, Hydroxy oder Niedrigalkoxy substituiert sein kann.

Unter Niedrigalkyl oder -alkoxy im Sinne der vorliegenden Erfindung werden solche Reste verstanden, die 1 - 5, vorzugsweise 1 - 3 C-Atome enthalten.

Z in Formel I bedeutet einen Glykosylrest, der bei den erfindungsgemäßen Verbindungen immer über das anomere Kohlenstoffatom an den Amidstickstoff gebunden ist, wobei unter Glykosylreste erfindungsgemäß besonders verstanden werden Mono-, Di- und Oligosaccharidreste, besonders Mono- und Disaccharide, in denen gegebenenfalls eine oder mehrere Hydroxylgruppen durch Aminogruppen, Acylamidogruppen,

Azidogruppen, Wasserstoff, Nitro, Thiolgruppen oder Niedrigalkoxy oder Halogen ersetzt sein können und die Glykosylreste auch in Form der entsprechenden Ulosen, Ulosederivate, Uronsäuren oder von Uronsäurederivaten vorliegen können.

Die Glykosylreste Z in Formel I liegen erfindungsgemäß bevorzugt in der Pyranosyl- oder der Furanosylform vor, wobei die betreffenden Monosaccharid-, Disaccharid- oder Oligosaccharidreste bevorzugt aus Tetrosen, Pentosen, Hexosen und Heptosen aufgebaut sind.

Erfindungsgemäße Beispiele für Monosaccharidreste sind Glucopyranosyl-, Galactopyranosyl-, Mannopyranosyl-, Glucofuranosyl-, Ribofuranosyl, Arabinopyranosyl- oder auch Lyxopyranosyl- oder auch D-Glycero-D-gluco-heptopyranosylreste. Als Beispiele für Di- und Oligosaccharidreste können genannt werden Maltosyl-, Maltotriosyl-, Maltotetraosyl-, Lactosyl-, Cellobiosyl-, Melibiosyl- oder 6-O-(α- oder ß-Ribofuranosyl)- Glucopyranosylreste, d.h. also Kohlenhydratsysteme, die aus Zuckern unterschiedlicher C-Zahl aufgebaut sind und bei denen die Zucker in der Pyranose und/oder Furanoseform vorliegen können. Die glykosidischen Bindungen zwischen den einzelnen Zuckerbausteinen können in der α- und/oder ß-Form vorliegen und die glykosidische Verknüpfung der einzelnen Zuckerbausteine kann von einem anomeren Kohlenstoffatom ausgehend sowohl über die primäre OH-Gruppe als auch über eine der sekundären Hydroxylgruppen des als Aglykon fungierenden Saccharidteils erfolgen.

Als Beispiele für Mono-, Di- und Oligosaccharidreste, in denen eine oder mehrere OH-Gruppen durch Aminogruppen, Acylamidogruppen, Azidogruppen, Wasserstoff, Nitro, Thiolgruppen, Niedrigalkoxy oder Halogen ersetzt sind, seien genannt 2-Acetylamido-2-desoxyglucopyranosyl , 2-Amino-2-desoxy-glucopyranosyl , 4-Azido-4-desoxy-glucopyranosyl , 4-Stearoylamido-4-desoxy-D-glucopyranosyl , 4-Dodecoylamido-4-desoxy-D-glucopyranosyl , 6-Hexadecanoylamido-6-desoxy-D-galactopyranosyl , 2,6-Diamino-2,6-didesoxyglucopyranosyl , 6,6'-Diamino-6,6'-didesoxymaltosyl , 6-Amino-6,6'didesoxylactosyl, 6-Desoxymannopyranosyl , 2-Desoxyribofuranosyl , Fucosyl, 5-Fluor-5-desoxyribofuranosyl , 6-O-Methylglucopyranosyl, 6-Desoxy-6-thio-glucopyranosyl, 3-Desoxy-3-nitrogalactopyranosyl.

Liegen die Glykosylreste in Form von Uronsäuren oder Uronsäurederivaten vor, so handelt es sich um Glykuronsäuren mit freier Carboxylgruppe oder mit durch Alkyl veresteter Carboxylgruppe oder um Glykuronamidderivate mit unsubstituiertem oder substituiertem Stickstoffatom. Beispiele für entsprechende Zucker sind Galacturonsäure, Glucuronsäuremethylester, Glucuronsäureamid oder auch N-Dodecylglucuronamid.

Die Verbindungen der Formel I enthalten mehrere chirale C-Atome und liegen als optisch reine Diasteromere oder als Diasteromerengemische vor. Die erfindungsgemäßen Verbindungen der Formel I sind also Carbonsäureamide

oder N-alkylierte bzw. N-Aralkylierte Carbonsäureamide, die jeweils am Amidstickstoff N-glykosidisch-d.h. also über das anomere Kohlenstoffatom gebunden- einen einfachen oder modifizierten Mono-, Di- oder Oligosaccacharidrest tragen.

Ganz besonders bevorzugt sind die durch die Ausführungsbeispiele belegten, insbesondere die der Beispiele 12, 13, 14, 15, 16, 18, 20, 24, 26, 34, 35, 36, 38, 40, 41, 43, 44, 46, 47, 49, 50, 51, 54, 55, 56, 57, 58 und 60, Verbindungen in ihrer Verwendung als Antirheumatika.

Die Verbindungen der Formel I können nach folgenden Verfahren hergestellt werden: Man setzt die von Z in Formel I umfaßten Zucker entweder in freier, d.h. ungeschützter Form, oder in Form geschützter, gegebenenfalls aktivierter Derivate zunächst mit einer Aminoverbindung $R_2-NH_2$, entweder in freier Form oder in Form eines geeigneten Säureadditionssalzes mit der vorstehend beschriebenen Bedeutung für $R_2$, um und acyliert anschließend das dabei erhaltene Glykosylamin mit einem - wie bei Acylierungsreaktionen üblich - aktivierten, an funktionellen Gruppen gegebenenfalls geschützten, Carbonsäurederivat, spaltet in dem so erhaltenen Reaktionsprodukt gegebenenfalls vorhandene Schutzgruppen ab und erhält auf diese Weise die Verbindungen der Formel I, die falls erforderlich, durch Chromatographie, Umkristallisieren, Extraktion o.ä. gereinigt werden können.

Das folgende Formelschema soll eine der bevorzugten Ausführungsformen der Darstellung von Verbindungen der Formel I beispielhaft erläutern:

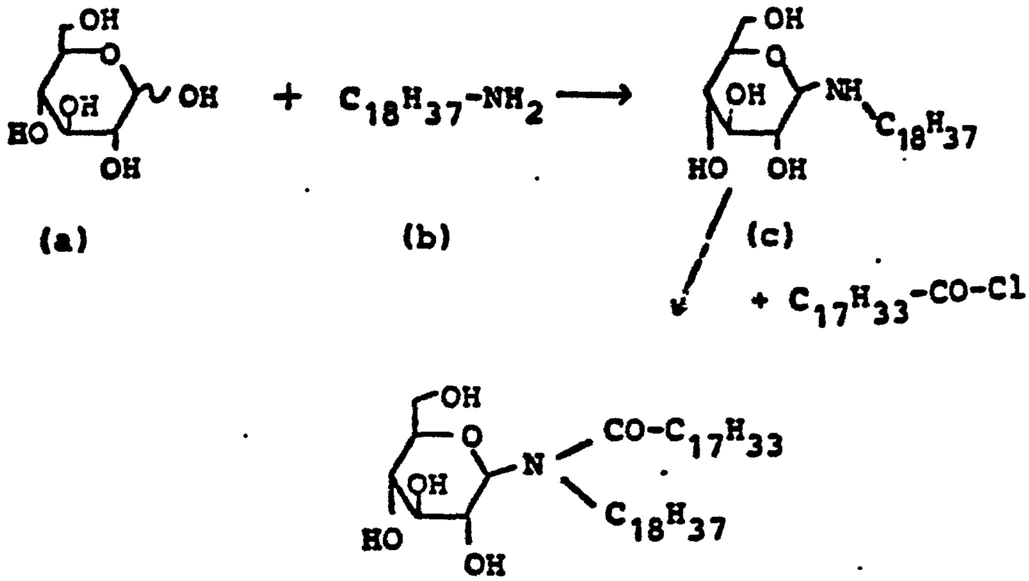


(a) (b) (c)

I

Im ersten Verfahrensschritt wird Glucose (a) mit Octadecylamin (b) zu N-Octadecyl-β-D-Glucopyranosylamin (c) umgesetzt, das im zweiten Verfahrensschritt mit Ölsäurechlorid zu N-Octadecyl-N-oleyl-β-D-glucopyranosylamin (I) acyliert wird.

Zum Gegenstand der Erfindung gehört auch die Verwendung der Salze der Verbindungen der Formel I als Mittel bei der Bekämpfung von Erkrankungen des rheumatischen Formenkreises. Dabei handelt es sich in erster Linie um üblicherweise pharmazeutisch verwendbare, nicht -toxische Salze, z.B. Alkalimetall- oder Ammonium- oder Erdalkalisalze.

Die Verbindungen der Formel I weisen wertvolle Eigenschaften, die sie bei der Bekämpfung von Erkrankungen des rheumatischen Formenkreises wertvoll machen,auf. Dies konnte anhand der nachstehend beschriebenen Versuchsanordnung gezeigt werden.

In einem vierwöchigen Versuch wurde die antiarthritische Wirkung von Verbindungen der Formel I am Modell der Freund'schen Adjuvans-Arthritis der Ratte untersucht. Der Wirkstoff wurde intraperitoneal täglich verabreicht. Wirkungskriterien waren: Körpergewichte, Schwellung der rechten und linken Hinterpfote, Sprunggelenkquerschnitt und hämatologische Parameter.

Die Wirkstoffe verringerten in der zweiten Versuchshälfte deutlich die nach einer Applikation vom Freund'schen Adjuvans normalerweise auftretende Körpergewichtsabnahme und hemmten dosisabhängig die Schwellung der rechten und linken Hinterpfote. Gegen Versuchsende verschwand die Schwellung der linken Pfote bei Ratten, die unverdünntes Präparat erhielten vollständig. Die unverdünnte Formulierung reduzierte den Sprunggelenkquerschnitt in der 2. - 4. Woche.

Die Verwendung der Verbindungen N-Glucosyl-N-octadecyl-dodecansäureamid und/oder N-Tetradecyl-N(2-amino-2-desoxy-D-glucopyranosyl)stearinsäureamid als Mittel bei der Bekämpfung von Erkrankungen des rheumatischen Formenkreises ist erfindungsgemäß bevorzugt.

Die pharmazeutischen Präparate der vorliegenden Erfindung sind vorzugsweise Tabletten oder Gelatinekapseln, welche die Wirkstoffe zusammen mit Verdünnungsmitteln, z.B. Laktose, Dextrose, Saccharose, Mannit, Sorbit, Cellulose und/oder Schmiermitteln, z.B. Kieselerde, Talk, Stearinsäure oder Salze davon, wie Magnesium- oder Calciumstearat, und/oder Polyethylenglykol, enthalten. Tabletten enthalten ebenfalls Bindemittel, z.B. Magnesiumaluminiumsilikat,

Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süssmittel. Injizierbare Präparate sind vorzugsweise isotonische wässrige Lösungen oder Suspensionen. Suppositorien, Salben oder Cremen sind in erster Linie Fettemulsionen oder -suspensionen. Ferner können die Substanzen in Liposomen, die sich tierexperimentell bewährt haben, inkorporiert werden. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1 % bis etwa 75 %, insbesondere von etwa 1 % bis 50 % der genannten Aktivstoffe.

Die oral applizierten Präparate der vorliegenden Erfindung können auch mit einem gegen Magensaft beständigen Überzug versehen werden.

Die erfindungsgemäßen Verbindungen können als Mittel bei der Bekämpfung von Erkrankungen des rheumatischen Formenkreises verwendet werden.

Die nachfolgenden Beispiele erläutern die vorstehend beschriebene Erfindung, sie sollen jedoch diese in ihrem Umfange in keiner Weise einschränken.

TP 68

Die Erfindung betrifft generell auch die Salze der Verbindungen I mit irgendwelchen anderen salzbildenden Gruppen, z.B. freien Carboxylgruppen, in erster Linie pharmazeutisch verwendbare, nicht toxische Salze, z.B. Metall- oder Ammoniumsalze und ihrer Verwendung als Antiarthritika oder entsprechenden Formulierungen bzw. Mitteln.

## Beispiele

Die Dünnschichtchromatographie (DC) erfolgte auf Kieselgel-DC-Fertigplatten (E. Merck, Darmstadt) und die präparativen Trennungen mit Kieselgel 60 (Merck, Darmstadt).

Laufmittelsysteme: System G $CH_2Cl_2/CH_3OH/15$ %iges Ammoniumhydroxid im Verhältnis 1/1/1, davon die Unterphase; System E $CH_2Cl_2/CH_3OH/20$ %iges Ammoniumhydroxid im Verhältnis 8/4/1; jeweils Volumenteile.

### Beispiel 1: N-D-Glucopyranosyl-Ölsäureamid

3 g 2,3,4,6-Tetra-O-acetyl-3,D-glucopyranosylamin wurden in 25 ml Tetrahydrofuran gelöst und nach Zugabe von 3,45 g Natriumcarbonat unter starkem Rühren und Kühlen bei 0°C tropfenweise mit 2,24 g Ölsäurechlorid gelöst und in 5 ml Tetrahydrofuran (THF) versetzt. Nach vollständiger Reaktion (Kontrolle durch Dünnschichtchromatographie = DC im System Toluol:Aceton = 4:1) wurde vom Niederschlag abfiltriert, das Filtrat im Vakuum eingedampft und getrocknet. Zur O-Deacetylierung löste man das so erhaltene Rohprodukt in 200 ml einer Lösung aus Methanol/Triethylamin/Wasser = 4:3:1 (Volumenteile) und beließ für 15 Stunden bei Raumtemperatur. Dann wurde im Vakuum eingedampft und der Rückstand an Kieselgel chromatographiert. Das auf diese Weise erhaltene Titelprodukt hatte einen Rf-Wert von 0.46.

<u>Beispiel 2:</u> N-Benzyl-ß-D-glucopyranosylamin

50 g D-Glucose wurden in 1000 ml heißem Ethanol gelöst und nach Zugabe von 89 g Benzylamin für 48 Stunden bei Raumtemperatur belassen. Dann wurde mit Eis gekühlt und das Produkt mit Petrolether gefällt. Es wurde abgesaugt, mit Ether gewaschen und im Vakuum getrocknet.

$^1$H-NMR in $CD_3OD$: $\delta$ = 7,33 breites Singulett Phenyl-H.

<u>Beispiel 3:</u> N-Benzyl-N-glucopyranosyl-acetamid

1 g der Verbindung aus Beispiel 2 wurde in 10 ml abs. Pyridin bei 0°C mit 6 ml Acetanhydrid bei Raumtemperatur acetyliert. Man arbeitete wie üblich auf und erhielt 1 g N-Acetyl-tetra-O-acetylderivat.

$^1$H-NMR in $CD\ Cl_3$: $\delta$ = 1,9 - 2,1 m 5 x $\underline{CH_3}$-CO-.

Zur O-Deacetylierung wurden 500 ml des Pentaacetats in abs. Methanol mit 10 % Natriummethylat deacetyliert und wie üblich aufgearbeitet. Das Produkt fiel als amorpher Feststoff an.

$^1$H-NMR in $CD_3OD$: $\delta$ = 7,1 - 7,4 Phenyl-H.

<u>Beispiel 4:</u> N-Dodecyl-ß-D-glucopyranosylamin

18 g Glucose wurden in 50 ml Ethanol bei 70°C gerührt, dann fügte man 18,5 g Dodecylamin zu, heizte noch bis zur

klaren Lösung, ließ auf Raumtemperatur abkühlen und saugte nach 20 Stunden von den ausgefallenen Kristallen ab. Man wusch mit Ethanol und Ether und trocknete im Vakuum. Ausbeute = 24 g.

Elementaranalyse: ($C_{18}H_{37}NO_5$ = 347):

ber. C = 62,2 %, H = 10,6 %, N = 4,0 %
gef. C = 62,2 %, H = 10,6 %, N = 4,2 %

Beispiel 5: N-Dodecyl-N-ß-D-glucopyranosyl-acetamid

Die Herstellung erfolgte analog Beispiel 3.

Elementaranalyse:
ber.: C = 61,7 %, H = 10,0 %, N = 3,6 %
gef.: C = 60,8 %, H = 9,9 %, N = 3,8 %

Beispiel 6: N-Glucopyranosyl-N-propyl-ölsäureamid

11 g N-Propyl-D-glucopyranosylamin wurden in 90 ml Tetrahydrofuran (THF) mit 21 g Soda gerührt, dann tropfte man 1 Äquivalent Ölsäurechlorid in 20 ml THF langsam unter Kühlen dazu. Nach vollständiger N-Acylierung (Kontrolle durch DC im Laufmittelsystem $CH_2Cl_2/CH_3OH$ = 13:1) wurde vom Niederschlag abgesaugt, mit THF nachgewaschen, die Filtrate im Vakuum eingedampft und der erhaltene Sirup an Kieselgel zur Nachreinigung chromatographiert. Entwicklung der Säule mit $CH_2Cl_2/CH_3OH$ = 15:1.

Die Fraktionen, die die Titelverbindung rein enthalten, wurden vereinigt. Das Lösungsmittel wurde im Vakuum entfernt. Ausbeute: 3,3 g.

Rf.-Wert: 0,34 in $CH_2Cl_2/CH_3OH$ = 15:1

$[\alpha]_D^{20}$ = + 7,5° (c=1,0 $CH_2Cl_2$)

<u>Beispiel 7:</u> N-Glucopyranosyl-N-hexyl-ölsäureamid

Die Herstellung erfolgte ausgehend von N-Hexyl-D-glucopyranosylamin wie im Beispiel 6 beschrieben. Säulenchromatographie mit $CH_2Cl_2/CH_3OH$ = 13:1.

Ausbeute: 9,2 g Reinprodukt.

Rf.-Wert = 0,38 in $CH_2Cl_2/CH_3OH$ = 13:1

$[\alpha]_D^{20}$ = + 5,8°C (c = 0,94 in $CH_2Cl_2$)

<u>Beispiel 8:</u> N-Glucopyranosyl-N-(n-3,3,3-trifluor-propyl)-ölsäureamid

3,6 g Glucose und 0,8 ml 0,5 N Salzsäure und 4,6 g n-3,3,3-Trifluorpropylamin wurden für 25 Minuten auf 75°C unter Rühren erhitzt. Nach Abkühlen kristallisierte das N-Glucosid aus, wurde mit Ether gewaschen und im Vakuum getrocknet.
Ausbeute: 4,1 g.

Die N-Acylierung mit Ölsäurechlorid erfolgte analog Beispiel 6. Säulenchromatographie mit $CH_2Cl_2/CH_3OH$ = 15:1. Ausbeute: 2,7 g.

$[\alpha]_D^{20}$ = + 7,6° (c = 1,0 in $CH_2Cl_2$)

Beispiel 9: N-(2-Ethyl-hexyl)-N-glucopyranosyl-ölsäureamid

Die Umsetzung von Glucose mit 2-Ethyl-hexylamin erfolgte analog Beispiel 8. Die N-Acylierung mit Ölsäurechlorid wurde analog Beispiel 6 durchgeführt. Säulenchromatographie mit $CH_2Cl_2/CH_3OH$ = 15:1.

Rf.-Wert der Titelverbindung: 0,44 in $CH_2Cl_2/CH_3OH$ = 15/1.

Beispiel 10: N-(3-Butoxy-propyl)-N-glucopyranosyl-ölsäureamid

Herstellung des N-Glycosids und N-Acylierung wie bei Beispiel 8 bzw. Beispiel 6 beschrieben.

Rf.-Wert: 0,29 Laufmittelsystem $CH_2Cl_2/CH_3OH$ = 10/1.

Beispiel 11: N-Dodecyl-N-glucopyranosyl-stearinsäureamid

100 g N-Dodecyl-ß-D-glucopyranosylamin aus Beispiel 4 wurden in 765 ml THF gelöst und in Gegenwart von 32 g

Triethylamin unter Kühlen tropfenweise mit 80 g Stearinsäurechlorid versetzt.

Zur Aufarbeitung wurde filtriert und das Lösungsmittel im Vakuum entfernt.

Ebenso wurde hergestellt N-Dodecyl-N-glucopyranosyl-Ölsäureamid.

Beispiel 12: N-Decyl-N-glucopyranosyl-Ölsäureamid

18 g D-Glucose und 50 ml Ethanol wurden bei 70°C mit 15,7 g Decylamin bis zur klaren Lösung gerührt. Dann ließ man auf Raumtemperatur abkühlen, saugte nach 4 Stunden die Kristalle ab und wusch mit Ethanol und Ether nach. Ausbeute: 20 g.

Diese wurden in 166 ml THF mit 22,6 g Soda gerührt. Dann tropfte man langsam 19 g Ölsäurechlorid in 20 ml THF bei 25°C zu. Nach einer weiteren Stunde wurde abgesaugt, das Filtrat im Vakuum zum Sirup eingedampft und das Rohprodukt säulenchromatographisch an Kieselgel mit dem Elutionsmittel $CH_2Cl_2/CH_3OH$ = 13/1 gereinigt.

Rf.-Wert der Titelverbindung = 0,53 in $CH_2Cl_2/CH_3OH$ = 13/2.

Beispiel 13: N-Glucopyranosyl-N-tetradecyl-Ölsäureamid

Herstellung analog Beispiel 12.

Säulenchromatographie mit dem Elutionsmittel $CH_2Cl_2/CH_3OH = 13/1$

$[\alpha]_D^{20} = + 9,6$ (c=1,0DMF)

Elementaranalyse:

ber. C = 70,3%, H = 11,3%, N = 2,16%

gef. C = 69,4%, H = 11,6%, N = 2,1%

Beispiel 14: N-Glucopyranosyl-N-hexadecyl-Ölsäureamid

Herstellung und Reinigung analog Beispiel 12

Rf.- Wert 0,25 Laufmittel $CH_2Cl_2/CH_3OH = 13/1$

Beispiel 15: N-Glucopyranosyl-N-octadecyl-Ölsäureamid

90 g D-Glucose und 135 g Octadecylamin wurden in 1.000 ml Propanol - 2 und 500 ml Wasser auf 50°C unter Rühren erhitzt bis eine klare Lösung entstanden war. Dann beließ man über Nacht bei Raumtemperatur. Nun wurde das Produkt abgesaugt, mit Alkohol und Ether gewaschen, getrocknet und zuletzt aus Ethanol/THF umkristallisiert. 10 g dieses N-Octadecyl-ß-D-glucopyranosylamins wurden in 80 ml THF aufgeschlämmt und nach Zusatz von 1o g Soda tropfenweise mit 7 g Ölsäurechlorid in 10 ml THF versetzt. Nach quantitativer Umsetzung (DC in $CH_2Cl_2/CH_3OH = 13/1$) wurde wie in Beispiel 12 beschrieben aufgearbeitet. Säulenreinigung mit Elutionsmittel $CH_2Cl_2/CH_3OH = 13/1$.
Rf -Wert = 0,35 Laufmittelsystem $CH_2Cl_2/CH_3OH = 9/1$.

**Beispiel 16:** N-Glucopyranosyl-N-Octadecyl-Stearinsäureamid

Herstellung analog Beispiel 6 aus N-Octadecyl-glucopyranosylamin und Stearinsäurechlorid.

Elementaranalyse:

ber.: C = 72,5%, H = 11,7%, N = 2,0%
gef.: C = 71,7%, H = 12,2%, N = 2,0%

**Beispiel 17:** N-Glucosyl-N-octadecyl-dodecansäureamid

Herstellung analog Beispiel 16 aus N-Octadecyl-ß-D-glucopyranosylamin und Dodecansäurechlorid.

$[\alpha]_D^{20}$ = + 8° (C= 1,0 Dioxan)

**Beispiel 18:** N-glucosyl-N-octadecyl-tetradecansäureamid

Herstellung analog Beispiel 16 aus N-Octadecyl-ß-D-glucopyranosylamin und Tetradecansäurechlorid

$[\alpha]_D^{20}$ = + 9,5° (C=1,0 DMF)

Elementaranalyse:

ber.: C = 71,8%, H = 11,7%, N = 2,1%
gef.: C = 71,3%, H = 11,9%, N = 1,9%

**Beispiel 20:** N-(2-Acetamido-2-desoxy-D-glucopyranosyl)-N-octadecyl-ölsäureamid

15 g N-Acetyl-D-glucosamin und 18,8 g Dodecylamin wurden für 3 Stunden in 50 ml Ethanol auf 80°C unter Rühren erhitzt. Dann wurde heiß vom Ungelösten abfiltriert, das Filtrat abgekühlt, das ausgefallene Produkt abgesaugt und mit Ethanol und Ether gewaschen, 2,2 g des so erhaltenen 2-Acetamido-2-desoxy-N-octadecyl-glucopyranosylamins wurden in 17 ml THF mit 2 g Soda gerührt. Dann versetzte man tropfenweise mit 1,45 g Ölsäurechlorid in 5 ml THF.

Aufarbeitung wie in Beispiel 6 beschrieben.

Säulenchromatographie mit dem Elutionsmittel $CH_2Cl_2/CH_3OH=20/1$.

$[\alpha]_D^{20} = + 9,2°$ (c= 0,56 $CH_3OH$)

Elementaranalyse:

ber.: C = 72,8%, H = 11,7%, N = 3,8%
gef.: C = 72,9%, H = 12,5%, N = 3,3%

**Beispiel 21:** N-Octadecyl-L-rhamnopyranosylamin

9 g L-Rhamnose und 13,5 g Stearylamin wurden in 100 ml Propanol - 2 und 50 ml Wasser bei 50°C solange gerührt, bis eine klare Lösung entstanden war. Nach 50 Stunden bei Raumtemperatur wurden die Kristalle abgesaugt, mit Ethanol und Ether gewaschen und im Vakuum getrocknet.
Ausbeute: 17,4 g
TP 68

Beispiel 22: N-Octadecyl-N-rhamnopyranosyl-ölsäureamid

7 g der Verbindung aus Beispiel 21 wurden wie in Beispiel 6 beschrieben mit Ölsäurechlorid acyliert. Säulentrennung in $CH_2Cl_2/CH_3OH$ = 13/1

Elementaranalyse:

ber.: C = 74,4%, H = 11,9%, N = 2,04%
gef.: C = 74,3%, H = 12,0%, N = 2,1%

Beispiel 23: N-Octadecyl-L-fucopyranosylamin

3,26 g L-Fucose und 5,38 g Stearylamin wurden in 20 ml Ethanol auf 70°C unter Rühren erhitzt, bis eine klare Lösung entstanden war. Man ließ abkühlen, saugte den Feststoff nach beendeter Kristallisation ab und wusch ihn mit Ethanol und Ether.

Ausbeute nach Trocknen in Vakuum: 4,4 g.

Beispiel 24: N-Fucopyranosyl-N-octadecyl-ölsäureamid

2,9 g der Verbindung aus Beispiel 23 wurden wie in Beispiel 6 beschrieben mit Ölsäurechlorid acyliert. Säulenchromatographie mit dem Elutionsmittel $CH_2Cl_2/CH_3OH$ = 15/1.

Ausbeute an Reinprodukt: 1,9 g

RF-Wert = 0,44 Laufmittelsystem wie bei Säulenchromatographie.

TP 68

**Beispiel 25:** N-ß-D-Arabinopyranosyl-N-octadecyl-ölsäureamid

7 g N-Octadecyl-ß,D-arabinopyranosylamin wurden wie in Beispiel 6 beschrieben mit Ölsäurechlorid acyliert. Säulenchromatographie mit Elutionsmittel $CH_2Cl_2/CH_3OH$ = 20/1.

Ausbeute an Reinprodukt: 2,3 g

Rf-Wert = 0,57 Laufmittel $CH_2Cl_2/CH_3OH$ = 15/1

$[\alpha]_D^{20}$ = + 20° (c = 1,03 $CH_2Cl_2$)

**Beispiel 26:** N-ß-D-Maltosyl-N-octadecyl-ölsäureamid

3,04 g N-Octadecyl-ß-D-maltosylamin wurden wie in Beispiel 6 beschrieben mit Ölsäurechlorid acyliert.

Säulenchromatographie in $CH_2Cl_2/CH_3OH$ = 10/1.

Rf -Wert: 0,24 Laufmittel $CH_2Cl_2/CH_3OH$ =8,1

$[\alpha]_D^{20}$ = + 22°C (c=0,5 $CH_3OH$)

**Beispiel 27:** N-(4-Azido-4-desoxy-D-glucopyranosyl)-N-octadecyl-dodecansäureamid

3,09 g 4-Azido-4-desoxy-D-glucose wurden in 30 ml Isopropanol und 15 ml Wasser gelöst und nach Zugabe von 4,05 g Octadecylamin auf 50°C erwärmt.

Die entstandene Lösung wurde über Nacht bei Raumtemperatur stehengelassen. Der entstandene Feststoff wurde abfiltriert, mit wenig Ethanol und Ether nachgewaschen und getrocknet.

2,3 g dieses Produktes wurden in 10 ml THF gelöst, mit 3 g Natriumcarbonat und 1,2 g Dodecansäurechlorid, gelöst in 15 ml THF, versetzt. Nach quantitativer Umsetzung wurde wie bei Beispiel 12 beschrieben aufgearbeitet.

Rf.-Wert: 0,27 in $CH_2Cl_2/CH_3OH$ = 4 : 1 (V/V)

Beispiel 28: N-(4-Acetamido-4-desoxy-D-glucopyranosyl)-N-octadecyl-ölsäureamid

3 g der Verbindung aus Beispiel 27 wurden in 30 ml Dioxan/Methanol = 2/1 und 3 ml Acetanhydrid in Gegenwart von 1,0 g Palladiumkohle (5%) bei Normaldruck hydriert. Nach Beendigung der Reaktion (Laufmittelsystem $CH_2Cl_2/CH_3OH$ = 3/1) wurde vom Katalysator abfiltriert und im Vakuum eingeengt.

Rf.-Wert: 0,18 ($CH_2Cl_2$/MeOH, 10:1 V/V)

Beispiel 29: N-(6-Desoxy-6-fluoro-D-glucopyranosyl)-N-octadecyl-ölsäureamid

18,2 g 6-Desoxy-6-fluor-D-glucose und 13,5 g Octadecylamin und 7 g Ölsäurechlorid wurden wie in Beispiel 15 beschrieben umgesetzt und aufgearbeitet.

Rf.-Wert: 0,30 in $CH_2Cl_2/CH_3OH$ = 9/1

**Beispiel 30:** N-(methyl-D-glucopyranosyluronato)-N-octadecyl-ölsäureamid

15 g D-Glucuronolacton wurden in 150 ml abs. Methanol gelöst und mit 3 ml 1 N Natriummethanolat-Lösung eine halbe Stunde bei Raumtemperatur stehengelassen. Anschließend wurde mit saurem Ionenaustauscher neutralisiert und eingedampft. Der entstandene Glucuronsäuremethylester wurde wie bei Beispiel 15 beschrieben zur Titelverbindung umgesetzt und aufgearbeitet.

Rf-Wert: 0,32 ($CH_2Cl_2/CH_3OH$ = 9:1, V/V)

**Beispiel 31:** N-(Glucuronopyranosyl)-N-octadecyl-ölsäureamid

2 g der in Beispiel 31 beschriebenen Verbindung wurden in 10 ml Dioxan gelöst und nach Zugabe von 5 ml 1 N Natronlauge 2 h am Rückfluß erhitzt. Nach dem Abkühlen wurde mit verd. Salzsäure neutralisiert, im Vakuum eingeengt und der Rückstand mit 20 ml Methanol/Dioxan = 1/1 gerührt. Anschließend wurde filtriert und das Filtrat zum Sirup eingeengt.

Rf.-Wert: 0,13 ($CH_2Cl_2/CH_3OH$ = 7:1, V/V)

Beispiel 32: N-(4-Amino-4-desoxy-D-glucopyranosyl)-N-octadecyl-laurinsäureamid

3 g der Verbindung aus Beispiel 27 wurden in 30 ml Dioxan/Methanol 2/1 in Gegenwart von 1,0 g Palladiumkohle (5%) hydriert. Nach Beendigung der Reaktion wurde vom Katalysator abfiltriert und i.Vac. eingeengt.

Rf.-Wert: 0,39 $CH_2Cl_2$/MeOH 5:1

Beispiel 33: N-(4-lauroylamido-4-desoxy-D-glucopyranosyl)-N-octadecyllaurinsäureamid

4,00 g in Beispiel 32 beschriebenen Verbindung wurden in 30 ml THF gelöst, mit 2,0 g Natriumcarbonat versetzt und mit 1,42 g Dodecansäurechlorid in 10 ml THF versetzt. Nach 30 Min. wurde mit Dichlormethan verdünnt, filtriert und das Filtrat i. Vac. eingeengt. Der Sirup wurde Säulenchromatographisch (Laufmittel Dichlormethan/Methanol = 15:1) gereinigt.

Rf.-Wert: 0,36 $CH_2Cl_2$/MeOH 10:1

Beispiel 34: N-Glucopyranosyl-N-octadecyl-palmitinsäureamid

Herstellung analog Beispiel 16 aus N-Octadecyl-glucopyranosylamin und Palmitinsäurechlorid.

Rf.-Wert: 0,36 $CH_2Cl_2$/MeOH 9:1

<u>Beispiel 35:</u> N-Octadecyl-N-glucopyranosyl-laurinsäureamid

Herstellung analog Beispiel 16 aus N-Octadecyl-glucopyranosylamin und Laurinsäurechlorid.

Rf-Wert: 0,35 $CH_2Cl_2/CH_3OH$ 9:1

<u>Beispiel 36:</u> N-Octadecyl-N-rhammopyranosyl-stearinsäureamid

Herstellung analog Beispiel 22 aus N-Octadecyl-rhammopyranosylamin und Stearinsäurechlorid.

Rf-Wert: 0,39 $CH_2Cl_2/CH_3OH$ 9:1

Beispiel 37: N-Octadecyl-(2-Amino-2-desoxy-D-glucopyranosyl) amin Hydrochlorid

6,45 g D-Glucosaminhydrochlorid wurden bei 60°C in 30 ml iso-Propanol und 10 ml Wasser gelöst und mit 12,1 g Stearylamin versetzt. Die entstandene klare Lösung wurde noch 10 min. weiter gerührt und dann auf Raumtemperatur abgekühlt. Das auskristalliisierte Produkt wurde abgesaugt und zunächst mit Ethanol/Wasser (5:2, v/v), dann mit Ethanol und schließlich mit Ether gewaschen. Der Rückstand wurde am Hochvakuum getrocknet.

Beispiel 38: N-Octadecyl-N-(2-dodecoylamido-2-desoxy-D-glucopyranosyl)-dodecansäureamid

4,6 g der in Beispiel 37 beschriebenen Verbindung wurden in 120 ml Tetrahydrofuran aufgeschlämmt und mit 22,6 g Natriumcarbonat versetzt. Zu der gerührten Suspension wurden 4,2 g Dodecansäurechlorid in 20 ml Tetrahydrofuran zugetropft. Der Ansatz wurde i.Vak. eingedampft, mit 50 ml Pyridin und 25 ml Acetanhydrid acetylisiert, auf Eiswasser gegossen, in Dichlormethan aufgenommen, die organische Phase wurde nacheinander mit verdünnter Salzsäure, gesättigter Natriumhydrogencarbonatlösung und dann mit Wasser gewaschen, über Natriumsulfat getrocknet und i.Vak. zum Sirup eingedampft. Der erhaltene Sirup wurde säulenchromatographisch gereinigt. (Laufmittel Toluol/Essigester = 10 : 1, v/v). Der erhaltene Feststoff (Schmp. 86°) wurde in abs. Methanol gelöst, mit 20 mg Natriummethoxid versetzt und 20 min unter Rückfluß erhitzt. Nach Beendigung der Reaktion wurde mit saurem Ionenaustauscher neutralisiert und i.Vak. eingedampft.

Schmelzpunkt: 78°C, Rf.-Wert: 0,64 in $CH_2Cl_2$/MeOH = 10/1 (v/v)

TP 68

**Beispiel 39:** N-Propyl-(2-amino-2-desoxy-D-glucopyranosyl) amin-Hydrochlorid

21,5 g Glucosaminhydrochlorid wurden in 17,7 g n-Propylamin aufgeschlämmt und auf 70° erwärmt, bis eine klare Lösung entstand. Beim Abkühlen auf Rt. fiel das Produkt aus.

**Beispiel 40:** N-Propyl-N-(2-ölsäureamido-2-desoxy-D-Glucopyranosyl)ölsäureamid

5,1 g der in Beispiel 39 beschriebenen Verbindung wurden in 100 ml Tetrahydrofuran aufgeschlämmt, mit 12,7 g Natriumcarbonat versetzt. Anschließend wurden 12 g Ölsäurechlorid in 20 ml Tetrahydrofuran zugetropft. Nach Beendigung der Reaktion wurde der Ansatz mit 50 ml Dichlormethan verdünnt, vom Natriumsalz abfiltriert, mit Wasser gewaschen, getrocknet über Natriumsulfat und i.Vak. eingedampft. Das erhaltene Sirup wurde säulenchromatographisch gereinigt (Laufmittel Dichlormethan/Methanol 15/1, v/v).

Rf.-Wert: 0,37 in $CH_2Cl_2$/MeOH 10:1

$[\alpha]_D^{20}$ = 17,9° (c = 1,02 in Dichlormethan)

**Beispiel 41:** N-Glucopyranosyl-N-tetradecyl-stearinsäureamid

Herstellung analog Beispiel 12 aus N-Tetradecyl-glucopyranosylamin und Stearinsäurechlorid.

Rf.-Wert: 0,25 in Toluol/Aceton 1:1

Beispiel 42: N-Dodecyl-N-(2-amino-2-desoxy-glucopyranosyl)-amin Hydrochlorid

46 g Dodecylamin wurden bei 60° aufgeschmolzen und unter Rühren mit 31 g Glucosaminhydrochlorid versetzt. Nach Abkühlen auf Rt. fiel das Produkt aus. Der Feststoff wurde dreimal mit Ether aufgerührt und abgesaugt und anschließend im Hochvakuum getrocknet.

Beispiel 43: N-Dodecyl-N-(2-stearoylamido-2-desoxy-D-glucopyranosyl)-stearinsäureamid

5 g der in Beispiel 42 beschriebenen Verbindung wurden in 100 ml Tetrahydrofuran aufgeschlämmt, mit 8,5 g Natriumcarbonat und 8 g Stearinsäurechlorid in 20 ml Tetrahydrofuran versetzt. Nach Beendigung der Reaktion wurde wie in Beispiel 40 beschrieben aufgearbeitet. Der erhaltene Rohsirup wurde aus Essigester kristallisiert.

Schmp. 67°; Rf.-Wert 0,42 in $CH_2Cl_2$/MeOH 10/1

Beispiel 44: N-Dodecyl-N-(2-laurinsäureamido-2-desoxy-D-glucopyranosyl)-laurinsäureamid

5 g der in Beispiel 42 beschriebenen Verbindung wurden wie bei Beispiel 43 beschrieben mit Laurinsäurechlorid umgesetzt.

Schmp. 67°; Rf.-Wert 0,42 in $CH_2Cl_2$/MeOH 10/1

Beispiel 45: N-Octadecyl-N-(galactopyranosyl)-amin

60 g D-Galactose wurden in 330 ml Isopropanol und 170 ml Wasser suspendiert und auf 50° erwärmt. Nach Zugabe von 90 g Stearylamin wurde so lange gerührt, bis alles Amin in Lösung gegangen war. Beim Erkalten kristallisierte das Glycosylamin aus. Der Feststoff wurde abgesaugt, nacheinander mit Ethanol und mit Ether gewaschen und i.Vak. getrocknet.

Beispiel 46: N-Octadecyl-N-(D-galactopyranosyl)-laurinsäureamid

Herstellung aus 8,4 g der in Beispiel 45 beschriebenen Verbindung und 4,4 g Dodecansäurechlorid analog Beispiel 11.

Rf.-Wert: 0,22 in Toluol-n-Propanol 4/1 (v/v)
$[\alpha]_D^{20}$ = 11,4 (c = 0,93 in Dichlormethan)

Beispiel 47: N-Tetradecyl-N-(D-galactopyranosyl)-ölsäureamid

Aus 30 g D-Galactose und 53 g Tetradecylamin wurde wie bei Beispiel 45 beschrieben N-Tetradecyl-N-(D-galactopyranosyl)-amin hergestellt. Das Galactosylamin wurde nach dem in Beispiel 11 beschriebenen Verfahren wird Ölsäurechlorid umgesetzt.

Rf.-Wert: 0,26 in Toluol/n-Propanol 4/1 (v/v)
$[\alpha]_D^{20}$ = 11° (c = 1,0 in Dichlormethan)

Beispiel 48: N-Octadecyl-N-mannopyranosyl-amin

20 g D-Mannose und 45 g Stearylamin wurden wie in Beispiel 45 beschrieben zum Glycosylamin umgesetzt.

Beispiel 49: N-Octadecyl-N-(D-mannopyranosyl)-laurinsäureamid

8,6 g der bei Beispiel 48 beschriebenen Verbindung wurden mit 4,4 g Dodecansäurechlorid wie bei Beispiel 11 beschrieben umgesetzt.

Rf.-Wert: 0,25 in Toluol/n-Propanol 4/1 (v/v)
$[\alpha]_D^{20}$ = 11,3° (c = 1,13 in Dichlormethan)

Beispiel 50: N-Octadecyl-N-(D-mannopyranosyl)-tetradecansäureamid

Herstellung aus der unter Beispiel 48 beschriebenen Verbindung und Tetradecansäurechlorid analog zu Beispiel 11.

Rf.-Wert: 0,26 in Toluol/n-Propanol 4/1 (v/v)
$[\alpha]^{20}$ = 9,9° (c = 1,0 in Dichlormethan)

Beispiel 51: N-Tetradecyl-N-(D-mannopyranosyl)-ölsäureamid

20 g D-Mannose und 35 g Tetradecylamin wurden wie bei Beispiel 45 beschrieben zum N-Tetradecyl-mannopyranosylamin umgesetzt. In einem zweiten Reaktionsschritt wurde das Glycosylamin (7,5 g) mit 6,0 g Ölsäurechlorid wie im Beispiel 11 beschrieben zum Glycosylamid umgesetzt.

Rf.-Wert: 0,29 in Toluol/n-Propanol 4/1 (v/v)
$[\alpha]_D^{20}$ = 10,8° (c = 1 in Tetrahydrofuran)

TP 68

Beispiel 52: 2-Dodecoylamido-2-desoxy-D-glucopyranose

55 g Dodecansäurechlorid wurden in 170 ml Tetrahydrofuran gelöst und unter starkem Rühren zu einer Lösung von 54 g D-Glucosamin-hydrochlorid in 330 ml wäßriger Natriumcarbonatlösung (20 %) getropft. Nach Beendigung der Säurechloridzugabe wurde noch eine Stunde weiter gerührt, dann wurde der Ansatz mit 500 ml Wasser versetzt und der Feststoff abgesaugt und mit Wasser gewaschen. Der Rückstand wurde aus iso-Propanol/Wasser 10/1 (v/v) umkristallisiert und im Hochvakuum getrocknet.

Beispiel 53: N-Dodecyl-N-(2-dodecoylamido-2-desoxy-D-glucopyranosyl)-amin

15 g der in Beispiel 52 beschriebenen Verbindung wurden mit 45 g Dodecylamin und 75 ml Ethanol versetzt und unter Rühren auf 70° erwärmt. Nachdem sich eine klare Lösung gebildet hatte, wurde auf Raumtemperatur abgekühlt und über Nacht kristallisiert. Der ausgefallene Feststoff wurde abgesaugt, einmal mit Ethanol und dreimal mit Ether gewaschen und i.Vak. getrocknet.

Beispiel 54: N-Dodecyl-N-(2-dodecoylamido-2-desoxy-D-glucopyranosyl)-stearinsäureamid

4 g der in Beispiel 53 beschriebenen Verbindung wurden in 100 ml Tetrahydrofuran gelöst und mit 4,8 g Natriumcarbonat versetzt. Zu dieser Suspension wurden unter

Rühren 3,45 g Stearinsäurechlorid in 20 ml Tetrahydrofuran gelöst zugetropft. Es wurde 30 min. weitergerührt, dann wurde mit 50 ml Dichlormethan verdünnt und vom Feststoff abgesaugt. Der Rückstand wurde mit Dichlormethan gewaschen. Die organischen Lösungsmittelphasen wurden vereinigt und i.Vak. eingeengt. Der erhaltene Sirup wurde chromatographisch gereinigt. (Laufmittel: Dichlormethan/Methanol 20/1 (v/v)).

Rf.-Wert: 0,55 in Dichlormethan/Methanol 10/1 (v/v)
$[\alpha]_D^{20} = 15,8°$ (c = 1,05 in Dichlormethan)

Beispiel 55: N-Dodecyl-N-(2-acetamido-2-desoxy-D-glucopyranosyl)-tetradecansäureamid

26 g N-Acetylglucosamin wurden in 100 ml Ethanol und 60 ml Wasser gelöst und auf 60° erwärmt. Es wurden 37 g Dodecylamin hinzugegeben und bis zur Einstellung einer klaren Lösung gerührt. Nach dem Abkühlen auf Raumtemperatur kristallisierte das Glycosylamin aus. Der Kristallbrei wurde abgesaugt, mit Ethanol und dann mit Ether gewaschen und i.Vak. getrocknet. 3 g des Feststoffes wurden in 50 ml Tetrahydrofuran aufgeschlämmt, mit 3,3 g Natriumcarbonat versetzt und mit 1,9 g Tetradecansäurechlorid in 10 ml Tetrahydrofuran versetzt. Nach Beendigung der Reaktion wurde mit 30 ml Dichlormethan verdünnt, filtriert und das Filtrat i.Vak. eingedampft. Der erhaltene Sirup wurde chromatographisch gereinigt (Laufmittel Dichlormethan/Methanol 20/1, (v/v)).

Rf.-Wert: 0,21 in Dichlormethan/Methanol 10/1 (v/v)

Beispiel 56: N-Dodecyl-N-(2-acetamido-2-desoxy-D-glucopyranosyl)-stearinsäureamid

3 g N-(2-Acetamido-2-desoxy)-dodecylamin, dessen Darstellung in Beispiel 55 beschrieben ist, wurden wie in Beispiel 55 beschrieben mit Stearinsäurechlorid umgesetzt.

Rf.-Wert: 0,23 in Dichlormethan/Methanol 10/1 (v/v)

Beispiel 57: N-Octadecyl-N-(2-acetamido-2-desoxy-D-glucopyranosyl)-tetradecansäureamid

Herstellung analog Beispiel 20 aus N-Acetylglucosamin, Stearylamin und Tetradecansäurechlorid.

Rf.-Wert: 0,25 in Toluol/iso-Propanol 4/1 (v/v)
$[\alpha]_D^{20}$ = 16,9° (c = 1 in Tetrahydrofuran)

Beispiel 58: N-Dodecyl-N-(D-mannopyranosyl)-stearinsäureamid

Herstellung aus D-Mannose, Dodecylamin und Stearinsäurechlorid analog Beispiel 11.

Rf.-Wert: 0,28 in Toluol/n-Propanol 4/1 (v/v)
$[\alpha]_D^{20}$ = 11,4° (c = 1 in Tetrahydrofuran)

Beispiel 59: N-Dodecyl-N-(D-galactopyranosyl)-stearinsäureamid

Herstellung aus D-Galactose, Dodecylamin und Stearinsäurechlorid analog Beispiel 11.

Rf.-Wert: 0,28 in Toluol/n-Propanol 4/1 (v/v)
$[\alpha]_D^{20}$ = 4,4° (c = 1 in Dichlormethan)

TP 68

Beispiel 60: N-Tetradecyl-N-(2-amino-2-desoxy-D-glucopyranosyl)stearinsäureamid-Hydroacetat

3,1 g 2-(Benzyloxycarbonylamino)-2-desoxy-D-Glucose wurden in 40 ml Dimethylformamid gelöst, mit 3,2 g Tetradecylamin versetzt und 4 Std. unter Wasserstrahlpumpenvakuum auf 80°C erwärmt. Die Mischung wurde auf Raumtemperatur abgekühlt, mit 50 ml Tetrahydrofuran verdünnt und in Gegenwart von 5 ml Ionenaustauscher SC 108 ($H^+$-Form) gerührt. Das Austauscherharz wurde abfiltriert und das Filtrat mit 1 g Natriumcarbonat und 3,3 g Stearinsäurechlorid versetzt. Nach fünfstündigem Rühren wurde von festen Bestandteilen abfiltriert und das Filtrat im Vakuum eingedampft. Der erhaltene Sirup wurde auf Kieselgel säulenchromatographisch gereinigt (Laufmittel Dichlormethan/Methanol = 20:1).

Das Hauptprodukt der Trennung wurde in 20 ml Dioxan, 20 ml Methanol und 20 ml Eisessig gelöst und in Gegenwart von 1 g Palladiumkohle (5 %) hydriert. Nach beendeter Wasserstoffaufnahme wurde der Katalysator abfiltriert, das Filtrat zum Sirup eingedampft und mehrfach zur vollständigen Entfernung der Essigsäure mit Ethanol coevaporiert.

$[\alpha]_D^{20}$ = + 10,2° (c = 0,95 in Tetrahydrofuran)

<u>Patentansprüche</u>

1) Verbindungen der allgemeinen Formel I

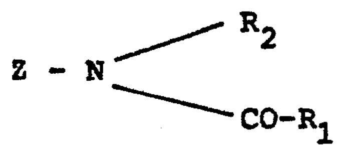

worin

Z einen über das anomere Kohlenstoffatom gebundenen Glycosylrest

$R_1$ Wasserstoff oder einen gegebenenfalls substituierten geradkettigen oder verzweigten, gesättigten oder ein- oder mehrfach ungesättigten Kohlenwasserstoffrest mit bis zu 30 C-Atomen, wobei dieser Rest auch durch O,N oder S unterbrochen sein kann und

$R_2$ Wasserstoff oder einen geradkettigen oder verzweigten, gesättigten oder ein- oder mehrfach ungesättigten Alkyl- oder Aralkylrest mit bis zu 30 C-Atomen, der durch O unterbrochen oder durch Sauerstoff enthaltende Gruppen oder Halogen substituiert sein kann

bedeuten mit der Maßgabe, daß $COR_1$ keine Acylgruppen mit 1-5 C-Atomen darstellt, wenn $R_2$ Alkyl mit 10-20 C-Atomen bedeutet, zur Verwendung bei der Bekämpfung von Krankheiten, insbesondere bei der Bekämpfung von Krankheiten des rheumatischen Formenkreises.

2) Verbindungen nach Anspruch 1, bei denen $R_1$ einen Alkyl- oder Alkenylrest mit 1-20 Kohlenstoffatomen darstellt, zur Verwendung bei der Bekämpfung von Krankheiten, insbesondere bei der Bekämpfung von Erkrankungen des rheumatischen Formenkreises.

3) Verbindungen nach den Ansprüchen 1 oder 2, bei denen $R_2$ einen Alkyl-oder Alkenylrest mit 1-20 C-Atomen darstellt, zur Verwendung bei der Bekämpfung von Krankheiten, insbesondere bei der Bekämpfung von Erkrankungen des rheumatischen Formenkreises.

4) Verbindungen nach den Ansprüchen 1-3, bei denen Z einen Monosaccharidrest, der gegebenenfalls durch eine Acylamidgruppe, wobei sich Acyl von einer Carbonsäure mit 1 bis 20 C-Atomen ableitet, substituiert ist, darstellt, zur Verwendung bei der Bekämpfung von Krankheiten, insbesondere bei der Bekämpfung von Erkrankungen des rheumatischen Formenkreises.

5) N-Octadecyl-N-D-glycopyranosyl-laurinsäureamid zur Verwendung bei der Bekämpfung von Erkrankungen des rheumatischen Formenkreises.

6) N-Octadecyl-N-D-glucopyranosyl-ölsäureamid zur Verwendung bei der Bekämpfung von Erkrankungen des rheumatischen Formenkreises.

7) N-Glucosyl-N-octadecyl-dodecansäureamid zur Verwendung bei der Bekämpfung von Erkrankungen des rheumatischen Formenkreises.

8) N-Tetradecyl-N-(2amino-2-desoxy-D-glucopyranosyl) stearinsäureamid zur Verwendung bei der Bekämpfung von Erkrankungen des rheumatischen Formenkreises.

9) Verwendung der Verbindungen gemäß den Ansprüchen 1-8 zur Bekämpfung von Krankheiten, insbesondere bei der Bekämpfung von Erkrankungen des rheumatischen Formenkreises.

10) Mittel für die Bekämpfung von Erkrankungen des rheumatischen Formenkreises, enthaltend eine oder mehrere Verbindungen der allgemeinen Formel I gemäß einem oder mehreren der Ansprüche 1 - 8.